# EUROPÄISCHE PATENTANMELDUNG

(11) **EP 3 167 863 A1**
(43) Veröffentlichungstag der Anmeldung: **17.05.2017**
(21) Anmeldenummer: 15196949.0
(22) Anmeldetag: 30.11.2015
(51) Int. Cl.: A61G 12/00

(54) **STATIONSWAGEN**

(30) Priorität: 13.11.2015 DE 202015106146 U
(71) Anmelder: Optiplan Gesellschaft für optische Planungsgeräte mit beschränkter Haftung, 40489 Düsseldorf (DE)
(72) Erfinder: Wagner, Kai, 40489 Düsseldorf (DE); Lerner, Sabine, 40489 Düsseldorf (DE); Godde, Dirk, 45665 Recklinghausen (DE)
(74) Vertreter: Stenger Watzke Ring

(57) **Zusammenfassung**

Die Erfindung betrifft einen Stationswagen, insbesondere einen Visiten- und Pflegewagen für Krankenhäuser, Pflegeheime und sonstige Einrichtungen des Gesundheitswesens, mit einem verfahrbar ausgebildeten Schrankteil (1), das einen einen Stauraum (4) bereitstellenden Korpus (2) und eine dem Korpus (2) oberseitig verschließende Abdeckplatte (3) aufweist, und das mit einer EDV-Anlage und einem daran angeschlossenen Monitor ausgerüstet ist. Um einen Stationswagen dahingehend weiterzuentwickeln, dass eine vereinfachte verwenderseitige Handhabung gestattet ist, wird mit der Erfindung vorgeschlagen, dass die Abdeckplatte (3) eine Ausnehmung (10) aufweist, in die der Monitor (11) mit seiner Frontscheibe bündig zu der von der Abdeckplatte (3) oberseitig bereitgestellten Abschlussfläche (13) eingesetzt ist.

## Beschreibung

Die Erfindung betrifft einen Stationswagen, insbesondere einen Visiten- und Pflegewagen für Krankenhäuser, Pflegeheimen und sonstige Einrichtungen des Gesundheitswesens, mit einem verfahrbar ausgebildeten Schrankteil, das einen einen Stauraum bereitstellenden Korpus und eine den Korpus oberseitig verschließende Abdeckplatte aufweist, und das mit einer EDV-Anlage und einem daran angeschlossenen Monitor ausgerüstet ist.

Die digitale Datenerfassung und Datenverarbeitung gewinnt auch im Kranken- und Pflegebereich zunehmend an Bedeutung. Zur Vermeidung von Übertragungsfehlern finden deshalb in verstärktem Maße Stationswagen Verwendung, die mit einer EDV Anlage ausgerüstet sind. Es ist auf diese Weise möglich, am Bett des Patenten Daten, zum Beispiel Anordnungen des Arztes bei der Visite, unmittelbar digital zu erfassen.

Stationswagen im Allgemeinen sowie solche, die mit einer EDV-Anlage ausgerüstet sind im Speziellen, sind aus dem Stand der Technik an sich bekannt. Eines gesonderten druckschriftlichen Nachweises bedarf es deshalb nicht. Nur beispielhaft seien die DE 20 2013 104 564 U1, die DE 20 2012 101 860 U1 und die DE 20 2011 051 973 U1 genannt, die jeweils einen gattungsgemäßen Stationswagen betreffen.

Die gemäß vorgenannter Druckschriften aus dem Stand der Technik bekannten Stationswagen haben sich im alltäglichen Praxiseinsatz bewährt. Es besteht gleichwohl Verbesserungsbedarf. Dabei liegt ein ständiges Bestreben darin, die Handhabung vorbekannter Stationswagen weiter zu verbessern.

Es ist deshalb die Aufgabe der Erfindung, einen Stationswagen der gattungsgemäßen Art dahingehend weiterzuentwickeln, dass eine vereinfachte verwenderseitige Handhabung gestattet ist.

Zur Lösung dieser Aufgabe wird mit der Erfindung vorgeschlagen ein Stationswagen der eingangs genannten Art, der sich dadurch auszeichnet, dass die Abdeckplatte eine Ausnehmung aufweist, in die der Monitor mit seiner Frontscheibe bündig zu der von der Abdeckplatte oberseitig bereitgestellten Abschlussfläche eingesetzt ist.

Gattungsgemäße Stationswagen verfügen über einen Monitor, der endseitig eines Monitorarms angeordnet ist. Von Nachteil bei dieser Anordnung ist, dass bei einem bestimmungsgemäßen Verfahren des Stationswagens durch Schieben der endseitig des Monitorarms angeordnete Monitor die Sicht für die den Stationswagen schiebende Person verdeckt. Dies wird verwenderseitig als unangenehm empfunden, da der Stationswagen quasi ohne freie Sicht geschoben wird, was dazu führen kann, dass etwaige Hindernisse übersehen werden, was dann zu ungewollten Kollisionen des Stationswagens mit diesen Hindernissen kommen kann. Solche Kollisionen können sowohl zur Beschädigung des Stationswagens als auch des Kollisionsobjektes führen. Im schlimmsten Fall kann es sogar zu Verletzungen kommen, nämlich dann, wenn es zu einer Kollision mit einer Person kommt.

Die erfindungsgemäße Ausgestaltung schafft hier Abhilfe. Denn im Unterschied zum Stand der Technik ist der vom Stationswagen bereitgestellte Monitor nicht endseitig eines Monitorarms angeordnet. Es ist vielmehr vorgesehen, dass der Monitor in die Abdeckplatte eingelassen ist, die den Korpus des Stationswagens verschließt. Die Abdeckplatte weist zu diesem Zweck eine Ausnehmung auf, in die der Monitor eingesetzt ist. Dabei schließt die Frontscheibe des Monitors in eingesetztem Zustand bündig mit der von der Abdeckplatte oberseitig bereitgestellten Abschlussfläche ab. Der Monitor ist mithin oberflächenbündig zur oberseitigen Abschlussfläche der Abdeckplatte ausgerichtet.

Die oberflächenbündige Anordnung des Monitors erbringt zwei wesentliche Vorteile. Zum einen wird eine hinterschnitt- und vorsprungfreie Gesamtoberfläche ausgebildet. Dies ist aus hygienischen Gründen von Vorteil, da Ansammelstellen für das Haftenbleiben von unerwünschten Verunreinigungen vermieden sind. Von Vorteil ist desweiteren, dass eine Gesamtablagefläche bereitgestellt wird, so dass die Abdeckplatte auch in herkömmlicher Weise als Ablage- oder Arbeitsfläche dienen kann.

Die Anordnung des Monitors in der Abdeckplatte ergibt nicht zuletzt den schon vorerläuterten Vorteil, dass bei einer Verschiebebewegung des Stationswagens keinerlei Sichtversperrung gegeben ist, wie dies bei aus dem Stand der Technik vorbekannten Stationswagen mit einem an einem Monitorarm angeordneten Monitor der Fall ist. Damit wird eine insgesamt vereinfachte Handhabung erreicht, die zudem auch aus sicherheitsrelevanten Aspekten von Vorteil ist.

Gemäß einem weiteren Merkmal der Erfindung ist vorgesehen, dass die Abdeckplatte zur Horizontalen geneigt ausgebildet ist. Dabei ist die Neigung derart ausgebildet, dass in Gebrauchsstellung des Stationswagens die frontseitige Randkante in Höhenrichtung unterhalb der rückseitigen Randkante der Abdeckplatte angeordnet ist. In Gebrauchsstellung ist die Abdeckplatte mithin in Richtung auf einen den Stationswagen in Verwendung nehmenden Verwender geneigt ausgebildet. Diese Neigung ist im Wesentlichen aufgrund zweier Gründe von Vorteil. Zum Einen werden unerwünschte Reflektionseffekte aufgrund einer bei einer Raumbeleuchtung typischerweise von oben stattfindenden Beleuchtung minimiert. Zum Anderen ist eine ergonomische Schreibhaltung gewährleistet, wenn die Abdeckplatte samt eingelassenem Monitor in herkömmlicher Weise als Ablage- oder Arbeitsplatte genutzt wird.

Gemäß einem weiteren Merkmal der Erfindung ist vorgesehen, dass die in Gebrauchsstellung des Stationswagen rückseitige Randkante konkav ausgebildet ist. Eine solche Ausgestaltung wirkt visuell ansprechender und ermöglicht es zudem, den Korpus mit einem ergänzend zum Stauraum vorgesehenen Zusatzraum auszurüsten, wie dies nachfolgend noch erläutert werden wird.

Gemäß einem weiteren Merkmal der Erfindung ist vorgesehen, dass der Korpus einen in Beschickungsrichtung des Stauraums dahinterliegenden und durch eine Zwischenwand vom Stauraum getrennten Zusatzraum aufweist, der eine Einrichtung zur Aufnahme eines Monitorarms beherbergt.

Gemäß dieser erfindungsgemäßen Weiterbildung ist einer vom Stauraum zur Aufnahme von Utensilien getrennter Zusatzraum vorgesehen. Zur Trennung von Stauraum und Zusatzraum dient eine Zwischenwand, so dass ein Zugriff auf den Zusatzraum nicht über den Stauraum möglich ist.

Der Zusatzraum dient der Beherbergung einer Einrichtung zur Aufnahme eines Monitors. Es ist so gestattet, den Stationswagen im Bedarfsfall mit einem Monitorarm auszurüsten. Ein solcher Monitorarm kann im Bedarfsfall mit einem zweiten Monitor bestückt sein. Gemäß dieser besonderen Ausführungsform verfügt der Stationswagen mithin über zwei Monitore, nämlich den ersten Monitor, der in schon vorbeschriebener Weise in die Abdeckplatte eingesetzt ist, sowie über einen zweiten Monitor, der endseitig eines vom Stationswagen getragenen Monitorarms angeordnet ist.

Diese Ausgestaltung hat den Vorteil, dass verwenderseitig in bestimmungsgemäßer Weise der von der Abdeckplatte aufgenommene Monitor in Einsicht genommen werden kann, es aber gleichzeitig die Möglichkeit über den zweiten Monitor gibt, die Anzeige des ersten Monitors auch einer im Bett liegenden Person, insbesondere einem Patienten über den zweiten Monitor zur Anzeige zu bringen. Medizinischem Personal wird es so einfacher, die Datenerfassung über den ersten Monitor vorzunehmen, gleichzeitig dem Patienten die Datenerfassung über die Anzeige des zweiten Monitors zu erklären und diesbezüglich gegebenenfalls zusätzliche Auskünfte zu geben. Gemäß dieser Ausführungsform müssen sich das medizinische Personal einerseits und Dritte, insbesondere der Patient oder die Patienten andererseits sich nicht einen gemeinsamen Monitor teilen, was eine Vereinfachung in der täglichen Handhabung darstellt.

Der zweite Monitor ist gemäß einem weiteren Merkmal der Erfindung um eine vertikal verlaufende Schwenkachse verschwenkbar am Monitorarm angeordnet, wobei der Verschwenkwinkel wenigstens 90°, vorzugsweise wenigstens 180° beträgt. Es kann so eine optimierte Ausrichtung des zweiten Monitors zur Einsichtnahme durch eine dritte Person vorgenommen werden. Zudem lässt sich der Monitor beim Verfahren des Stationswagens so verschwenken, dass eine nur minimierte Sichtbeeinträchtigung gegeben ist.

Zumindest der erste Monitor ist gemäß einem weiteren Merkmal der Erfindung als touchscreenfähiger Monitor ausgebildet. Bevorzugt ist es indes, beide Monitore in Form jeweils eines Touchscreens auszubilden. Dies ist insbesondere aus Gründen der vereinfachten Handhabung bei der Dateneingabe von Vorteil, sowie auch hinsichtlich hygienischer Aspekte, da auch bei einer Touchscreeneingabe Handschuhe getragen werden können und es darüber hinaus möglich ist, mit entsprechenden Desinfektionsmitteln die Oberflächen der Monitore als auch der Abdeckplatte keimfrei zu halten.

Die Ausrüstung des Stationswagens mit einer Einrichtung zur Aufnahme eines Monitors ist auch insofern von Vorteil, als dass im Bedarfsfall eine Nachrüstung vorgenommen werden kann, ebenso wie eine Abrüstung, je nachdem für welchen Verwendungszweck der Stationswagen zukünftig eingesetzt wird. Dabei gestattet es die Nachrüstbarkeit beziehungsweise die Abrüstbarkeit, den Einsatzzweck des Stationswagens auch ohne Einbuße in der Handhabung ändern zu können.

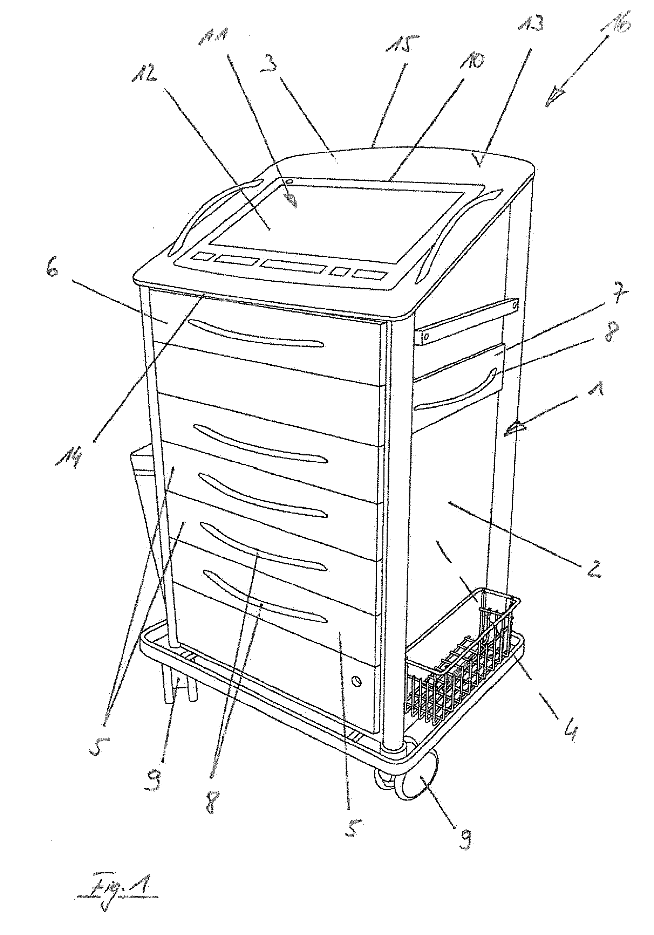

Weitere Merkmale und Vorteile der Erfindung ergeben sich aus der nachfolgenden Beschreibung anhand der einzigen Figur 1, die in schematisch perspektivischer Darstellung einen erfindungsgemäßen Stationswagen 16 zeigt.

Der erfindungsgemäße Stationswagen 16 verfügt in an sich bekannter Weise über ein Schrankteil 1. Dieses weist einen Korpus 2 auf, der in Höhenrichtung oberseitig von einer Abdeckplatte 3 verschlossen ist.

Der Korpus 2 stellt einen Stauraum 4 zur Aufnahme von Utensilien bereit, bei welchen Utensilien es sich um Verbandmaterialien, Scheren, Beutel, Handschuhe und/oder dergleichen handeln kann. Für einen besseren Zugriff auf die Utensilien sind bevorzugterweise Schubladen 5 vorgesehen. Es können auch seitlich ausziehbare Schubladen 7 vorgesehen sein, wie in Fig. 1 dargestellt. Dabei verfügen die Schubladen 5 und 7 zwecks Handhabung in an sich bekannter Weise über entsprechende Griffe 8.

Der Stationswagen 16 ist darüber hinaus mit einer in der Figur nicht näher dargestellten EDV-Anlage ausgerüstet. Zur Bedienung der EDV-Anlage sind eine Tastatur und/oder eine Mouse vorgesehen, die in einer weiteren Schublade 6 untergebracht sein können. Der Stationswagen 16 ist als verfahrbarer Wagen ausgebildet, zu welchem Zweck der Korpus 2 unterseitig über Rollen 9 verfügt.

Die Abdeckplatte 3 verfügt erfindungsgemäß über eine Ausnehmung 10. In diese ist ein Monitor 11 eingesetzt, und zwar derart, dass der Monitor 11 mit seiner Frontscheibe 12 bündig zu der von der Abdeckplatte 3 oberseitig bereitgestellten Abschlussfläche 13 ausgerichtet ist. Der Monitor 11 und die Abdeckplatte 3 schließen mithin oberseitig oberflächenbündig ab.

Die Abdeckplatte 3 ist samt darin eingesetztem Monitor 11 zur Horizontalen geneigt, und zwar in Richtung auf einen in Gebrauchsstellung des Stationswagen 16 vor dem Stationswagen 16 stehenden Verwender. Die frontseitige Randkante 14 der Abdeckplatte 3 ist mithin in Höhenrichtung unterhalb der rückseitigen Randkante 15 der Abdeckplatte 3 angeordnet. Bevorzugterweise ist die rückseitige Randkante 15 der Abdeckplatte 3 konkav ausgebildet, wie sich dies ebenfalls aus Fig. 1 ergibt.

Gemäß einer Weiterentwicklung der Erfindung kann vorgesehen sein, dass der Stationswagen 16 über einen zweiten Monitor verfügt. Dieser ist bevorzugterweise endseitig eines Monitorarms angeordnet. Die Anordnung eines solchen zweiten Monitors kann in vorteilhafterweise im Wege der Nachrüstung erfolgen. Zu diesem Zweck verfügt der Stationswagen 16 über einen Zusatzraum, der eine Einrichtung zu Aufnahme eines Monitorarms beherbergt. Dieser Zusatzraum ist in Beschickungsrichtung des vom Korpus 2 bereitgestellten Stauraums 4 hinter dem Stauraum 4 liegend angeordnet. Der Stauraum 4 und der Zusatzraum sind mittels einer Trennwand voneinander getrennt ausgebildet, so dass ein direkter Zugriff auf den Zwischenraum über den Stauraum 4 nicht möglich ist.

Sofern dies je nach Anwendungsfall gewünscht ist, ist die Abdeckplatte 3 mit einer Bohrung für ein Hindurchführen des Monitorarms auszurüsten. Alsdann kann der Monitorarm durch diese Bohrung in den Zusatzraum eingeführt werden, so dass mittels der vom Zusatzraum bereitgestellten Einrichtung zur Aufnahme eines Monitorarms eine Lagefixierung und -sicherung des Monitorarms vorgenommen werden kann.

Der zweite Monitor ist bevorzugterweise um eine vertikal verlaufende Schwenkachse verschwenkbar, wenigstens um 90°, vorzugsweise um wenigstens 180°. Es ist so gestattet, den Monitor in Richtung auf eine beispielsweise in einem Krankenhaus- oder Pflegebett liegende Person auszurichten, während das den Stationswagen 16 nutzende medizinische Personal nach wie vor einen ungehinderten Einblick und Zugriff auf den von der Abdeckplatte 3 aufgenommenen Monitor 11 hat.

### Bezugszeichenliste

- 1: Schrankteil
- 2: Korpus
- 3: Abdeckplatte
- 4: Stauraum
- 5: Schublade
- 6: Schublade
- 7: seitliche Schublade
- 8: Handgriff
- 9: Rolle
- 10: Ausnehmung
- 11: Monitor
- 12: Frontscheibe
- 13: Abschlussfläche
- 14: frontseitige Randkante
- 15: rückseitige Randkante
- 16: Stationswagen

## Patentansprüche

1. Stationswagen, insbesondere Visiten- und Pflegewagen für Krankenhäuser, Pflegeheime und sonstige Einrichtungen des Gesundheitswesens, mit einem verfahrbar ausgebildeten Schrankteil (1), das einen einen Stauraum (4) bereitstellenden Korpus (2) und eine den Korpus (2) oberseitig verschließende Abdeckplatte (3) aufweist, und das mit einer EDV-Anlage und einem daran angeschlossenen Monitor (11) ausgerüstet ist,
**dadurch gekennzeichnet,**
**dass** die Abdeckplatte (3) eine Ausnehmung (10) aufweist, in die der Monitor (11) mit seiner Frontscheibe bündig zu der von der Abdeckplatte (3) oberseitig bereitgestellten Abschlussfläche (13) eingesetzt ist.

2. Stationswagen nach Anspruch 1, **dadurch gekennzeichnet, dass** die Abdeckplatte (3) zur Horizontalen geneigt ausgebildet ist, wobei die in Gebrauchsstellung des Stationswagens (1) frontseitige Randkante (14) in Höhenrichtung unterhalb der rückseitigen Randkante (15) der Abdeckplatte (3) angeordnet ist.

3. Stationswagen nach Anspruch 2, **dadurch gekennzeichnet, dass** die in Gebrauchsstellung des Stationswagens (16) rückseitige Randkante (15) konkav ausgebildet ist.

4. Stationswagen nach einem der vorhergehenden Ansprüche, **dadurch gekennzeichnet, dass** der Korpus (2) einen in Beschickungsrichtung des Stauraums (4) dahinterliegenden und durch eine Zwischenwand vom Stauraum (4) getrennten Zusatzraum aufweist, der eine Einrichtung zur Aufnahme eines Monitorarms beherbergt.

5. Stationswagen nach Anspruch 4, **gekennzeichnet, durch** einen zweiten an die EDV-Anlage angeschlossenen Monitor, der von einem von der Einrichtung zur Aufnahme eines Monitorarms aufgenommenen Monitorarm getragen ist.

6. Stationswagen nach Anspruch 5, **dadurch gekennzeichnet, dass** der zweite Monitor um eine vertikal verlaufende Schwenkachse verschwenkbar am Monitorarm angeordnet ist.

7. Stationswagen nach Anspruch 6, **dadurch gekennzeichnet, dass** der Verschwenkwinkel wenigstens 90°, vorzugsweise wenigstens 180° beträgt.

8. Stationswagen nach Anspruch 1, **dadurch gekennzeichnet, dass** der erste Monitor (11) als touchscreenfähiger Monitor ausgebildet ist.
